# EUROPEAN PATENT SPECIFICATION

(11) **EP 2 411 533 B1**
(45) Date of publication and mention of the grant of the patent: **23.03.2016**
(21) Application number: 10709859.2
(22) Date of filing: 25.03.2010
(51) Int. Cl.: C12Q 1/68

(54) **Genetic severity markers in multiple sclerosis**
Genetische Marker für die Schwere von multipler Sklerose
Marqueurs génétiques de gravité de la sclérose en plaques

(30) Priority: 27.03.2009 EP 09156487; 31.03.2009 US 165141 P
(43) Date of publication of application: 01.02.2012
(73) Proprietor: Merck Serono S.A., 1267 Coinsins (CH)
(72) Inventor: ABDERRAHIM, Hadi, F-01220 Divonne les Bains (FR); WOJCIK, Jérôme, F-01220 Divonne Les Bains (FR); ESPOSITO, Federica, I-24039 Sotto II Monte Giovanni XXIII (BG) (IT); DEBAILLEUL, Virginie, F-01630 Peron (FR)
(74) Representative: Merck Serono S.A. Intellectual Property
(86) International application number: PCT/EP2010/053871
(87) International publication number: WO 2010/127906

(56) References cited:
- WO-A2-2006/076641
- VAN VEEN ET AL: "CCL5 and CCR5 genotypes modify clinical, radiological and pathological features of multiple sclerosis" JOURNAL OF NEUROIMMUNOLOGY, ELSEVIER SCIENCE PUBLISHERS BV, XX, vol. 190, no. 1-2, 1 October 2007 (2007-10-01), pages 157-164, XP022350631 ISSN: 0165-5728
- FUKAZAWA TOSHIYUKI ET AL: "CTLA-4 gene polymorphism may modulate disease in Japanese multiple sclerosis patients" JOURNAL OF THE NEUROLOGICAL SCIENCES, vol. 171, no. 1, 1 December 1999 (1999-12-01), pages 49-55, XP002567285 ISSN: 0022-510X
- MANN C L A ET AL: "Interleukin 1 genotypes in multiple sclerosis and relationship to disease severity" JOURNAL OF NEUROIMMUNOLOGY, vol. 129, no. 1-2, August 2002 (2002-08), pages 197-204, XP002567286 ISSN: 0165-5728
- AMBROSIUS MICHAEL ET AL: "The xylosyltransferase Iota gene polymorphism c.343G>T (p.A115S) is associated with decreased serum glycosaminoglycan levels." CLINICAL BIOCHEMISTRY JAN 2009, vol. 42, no. 1-2, January 2009 (2009-01), pages 1-4, XP002567288 ISSN: 1873-2933
- LEE SUNG-UK ET AL: "N-glycan processing deficiency promotes spontaneous inflammatory demyelination and neurodegeneration." THE JOURNAL OF BIOLOGICAL CHEMISTRY 16 NOV 2007, vol. 282, no. 46, 16 November 2007 (2007-11-16), pages 33725-33734, XP002567287 ISSN: 0021-9258
- BRYNEDAL B ET AL: "MGAT5 alters the severity of multiple sclerosis" JOURNAL OF NEUROIMMUNOLOGY, ELSEVIER SCIENCE PUBLISHERS BV, XX LNKD- DOI:10.1016/J.JNEUROIM.2010.01.003, vol. 220, no. 1-2, 30 March 2010 (2010-03-30), pages 120-124, XP026939091 ISSN: 0165-5728 [retrieved on 2010-02-01]

### SEQUENCE LISTING

<110> Merck Serono S.A.
<120> Genetic Severity Markers in Multiple Sclerosis
<130> P 09/042 WO/PCT
<150> EP 09156487.2
   <151> 2009-03-27
<150> US 61/165,141
   <151> 2009-03-31
<160> 14
<170> PatentIn version 3.5
<210> 1
   <211> 33
   <212> DNA
   <213> Homo sapiens
<220>
   <221> variation
   <222> (17)..(17)
   <223> y is c or t
<400> 1
   cattgcaact catctayacc tgtaactctt gtt 33
<210> 2
   <211> 33
   <212> DNA
   <213> Homo sapiens
<220>
   <221> variation
   <222> (17)..(17)
   <223> y is c or t
<400> 2
   tagccgttgt tgtccayctc ctccaataga atg 33
<210> 3
   <211> 33
   <212> DNA
   <213> Homo sapiens
<220>
   <221> variation
   <222> (17)..(17)
   <223> r is a or g
<400> 3
   ggctggctgt cccgccraac aaagagcctg gat 33
<210> 4
   <211> 33
   <212> DNA
   <213> Homo sapiens
<220>
   <221> variation
   <222> (17)..(17)
   <223> m is a or c
<400> 4
   ttgaccagcc ttatcamatc tgactgtatt tcc 33
<210> 5
   <211> 33
   <212> DNA
   <213> Homo sapiens
<220>
   <221> varitation
   <222> (17)..(17)
   <223> r is g or a
<400> 5
   cccaaagatg ccggacrgat accccaagag gtg 33
<210> 6
   <211> 33
   <212> DNA
   <213> Homo sapiens
<220>
   <221> variation
   <222> (17)..(17)
   <223> w is t or a
<400> 6
   gtttataaaa actctcwgaa acctcaaaga aca 33
<210> 7
   <211> 33
   <212> DNA
   <213> Homo sapiens
<220>
   <221> variation
   <222> (17)..(17)
   <223> r is g or a
<400> 7
   gaatcaggtt ctgatcraga tccacaaatt tta 33
<210> 8
   <211> 33
   <212> DNA
   <213> Homo sapiens
<220>
   <221> variation
   <222> (17)..(17)
   <223> y is t or c
<400> 8
   gcaattaccg gtaagcyatg agagtagtgg ggg 33
<210> 9
   <211> 33
   <212> DNA
   <213> Homo sapiens
<220>
   <221> variation
   <222> (17)..(17)
   <223> r is g or a
<400> 9
   tgttgctgac aattaarcca catagcattt ata 33
<210> 10
   <211> 33
   <212> DNA
   <213> Homo sapiens
<220>
   <221> variation
   <222> (17)..(17)
   <223> m is a or c
<400> 10
   ttgcatcttt gggttamggc tctgctgccc ttg 33
<210> 11
   <211> 33
   <212> DNA
   <213> Homo sapiens
<220>
   <221> variation
   <222> (17)..(17)
   <223> r is a or g
<400> 11
   agtccctaag tgccacraat gaaaagaaga ctc 33
<210> 12
   <211> 33
   <212> DNA
   <213> Homo sapiens
<220>
   <221> variation
   <222> (17)..(17)
   <223> s is g or c
<400> 12
   tttaattccc cacaaasagc tgagtggctc ttg 33
<210> 13
   <211> 33
   <212> DNA
   <213> Homo sapiens
<220>
   <221> variation
   <222> (17)..(17)
   <223> r is g or a
<400> 13
   ggaaaacaaa ttttccrctt ctaaggctgt taa 33
<210> 14
   <211> 33
   <212> DNA
   <213> Homo sapiens
<220>
   <221> variation
   <222> (17)..(17)
   <223> y is c or t
<400> 14
   tgaatgagat aattcaygtg aggctcttag aaa 33

## Description

### Field of the invention

The present invention relates to the use of SNPs to identify an association with the severity of Multiple Sclerosis (MS) in a subject.

### Background of the invention

Multiple Sclerosis (MS) is a chronic inflammatory and demyelinating disease of the Central Nervous System (CNS), often starting in early adulthood. MS is considered a complex disease, since multiple genetic and non-genetic factors are likely to combine to influence the risk to disease. The evidence for a role of genetic factors is compelling and is supported by twin, half-sibling and adoptee studies. Whereas MS usually starts with a relapsing-remitting course (RR), most patients later enter a secondarily progressive phase (SP) while others, often with a later onset, may enter directly into primary progression (PP).

Genome scans have excluded the presence of a major susceptibility locus in MS apart from the HLA class II region, and failed to reveal more than a few putative susceptibility *loci* ¹⁻³. Within the HLA gene complex, associations with several alleles of HLA-DRB1 have been indicated ⁴, whereas some evidence also suggests an independent factor for risk of MS in the HLA class I region ⁵⁻⁷. Very recently, evidence supporting an importance of the IL7Ra gene in MS is mounting ⁸⁻¹⁰. However, it is clear that other genetic risk factors remain to be identified.

Susceptibility to MS is unequivocally a complex genetic trait. Clinical course and outcome of MS differ widely and it seems likely that while some genes may be involved in the induction of the disease, others may have a role in influencing the disease severity ^{11,12}. Severity in MS is assessed as development of disability as a function of duration of disease but may be complicated by the fact that the rate of progression differs from time to time and that patients may also show periods of improvement. The most widely used method of clinical assessment of MS severity is based on the Expanded Disability Status Scale (EDSS ¹³). Traditionally, the Progression Index (PI = EDSS score / duration in years) has been widely used but is hampered by the reasons mentioned above. More recently the MS Severity Score (MSSS) has been proposed as a novel approach, relating scores on the EDSS to the distribution of disability in patients with comparable disease durations, partly compensating for the weaknesses of the PI ¹⁴.

Several candidate genes have been tested for a possible association with MS severity (see ¹⁵ for review). Most of them displayed no evidence of genetic association with MS prognosis: apolipoprotein ε (APOE, see ¹⁶ for review), spinocerebellar ataxia 2 (SCA2 ¹⁷), brain-derived neurotrophic factor (BDNF ¹⁸), toll-like receptor 4 (TLR4 ¹⁹), osteopontin ²⁰, cytotoxic T lymphocyte associated 4 (CD152 or CTLA4) and CD28 ²¹, and chemokine CC receptor 5 (CCR5) and HLA-DRB1*1501 ²². Only a handful of *loci* have been reported to be associated with the clinical outcome of MS: the interleukin-1 locus on chromosome 2q12-14 contains 3 genes (IL-1α, IL-1β and IL-1 receptor antagonist IL-1RN) in which 6 sites, 5 single nucleotide polymorphisms (SNPs) and one variable number tandem repeat (VNTR), were reported to be associated with severity measured by EDSS graded in three severity categories ²³ ; in the interleukin-10 promoter, two microsatellite markers were reported as differentially represented between mild (PI < 0.5) and severe (PI > 0.5) disease progression categories ²⁴ ; two SNPs have been found associated with MS categories (relapsing remitting - RR vs. primary progressive - PP) but not with prognosis measured by MSSS in the ADAMTS14 gene ²⁵ ; and disease incidence and severity have been shown to be increased in CD59a-deficient in MOG-EAE murine model ²⁶. However, these studies were based on limited numbers of individuals and none was replicated. In addition, all these studies used categorical approaches to detect association with severity: the patients are categorized in mild/moderate/severe or mild/severe MS forms by choosing cutoff thresholds on the lesion volumes, the EDSS, the PI or the MSSS scales, and frequencies of alleles and genotypes are compared between categories.

WO2006/076641 hows three SNPs of MGAT5 identified in MS patients and determination of the SNPs in both alleles. D4 also discloses that the severity of the MS disease is inversely correlated with MGAT5 Finally, D4 discloses the treatment of MS patients with beta-interferon, eg Avonex or Rebiff

AMBROSIUS M ET AL: "CLINICAL BIOCHEMISTRY, vol. 42, no. 1-2, January 2009 (2009-01), pages 1-4 discloses genotyping of both alleles of three SNPs in the XYLT1 gene by PCR and restriction digestion and that a SNP in the XYLT1 gene is linked to a lower glycan production.

In view of the significance of MS there is a need to identify markers, in particular genetic markers, useful in MS patients. In particular there exists a need to identify genetic markers useful predicting susceptibility and in particular severity of the disease MS.

### Summary of the invention

The present invention in one aspect is directed to a method for predicting the severity of MS by determining the type of nucleotide in SNP rs2059283 and/or rs12927173 in one or both of the alleles of the diallelic as defined in claim 1.

Disclosed are one or more SNPs selected from the group consisting of SNPs rs3814022, rs4953911, rs2059283, rs12927173, rs2495725, rs1343522, rs4573623, rs333548, rs10508075, rs2839580, rs2495725, rs3814022, rs1078922, rs4315313, SNPs in Linkage Disequilibrium (LD) with one or more of these SNPs for use in predicting in an individual the severity of the disease Multiple Sclerosis.

In yet another aspect the invention relates to interferon-beta for use in for treating Multiple Sclerosis in an individual as defined in claim 7, the method comprising the steps of a. applying a method as described above to a sample of an individual in vitro; b. treating said individual identified to exhibit one or more of the markers described above and which individual has been identified to exhibit a certain level of severity of the disease Multiple Sclerosis.

### Detailed description of the invention

In the following the invention will be described in more detail wherein the examples are intended to illustrate the invention without being construed to be limiting the scope of the invention.

### Brief description of the Tables and Figures:

### Figure 1. MSSS distribution.

Histogram of the MS Severity Score distribution over the 1,040 MS patients.

### Figure 2. FDR (False Discovery Rate) estimation of severity associations.

The FDR was estimated with 10,000 rounds of MSSS shuffling and plotted against the number of selected positives *R*, for *R* ≤ 100 (thick line). This curve gives the estimated proportion of false-positives for a given number of positives (*e*.*g*. 90% of the 40 most likely associated SNPs (*R* ≤ 40) are estimated to be false-positives) or the number of positives for a given false-discovery rate (e.g. only one SNP is selected at 40% FDR threshold). Dashed lines represent the boundaries of the 95% estimation confidence interval.

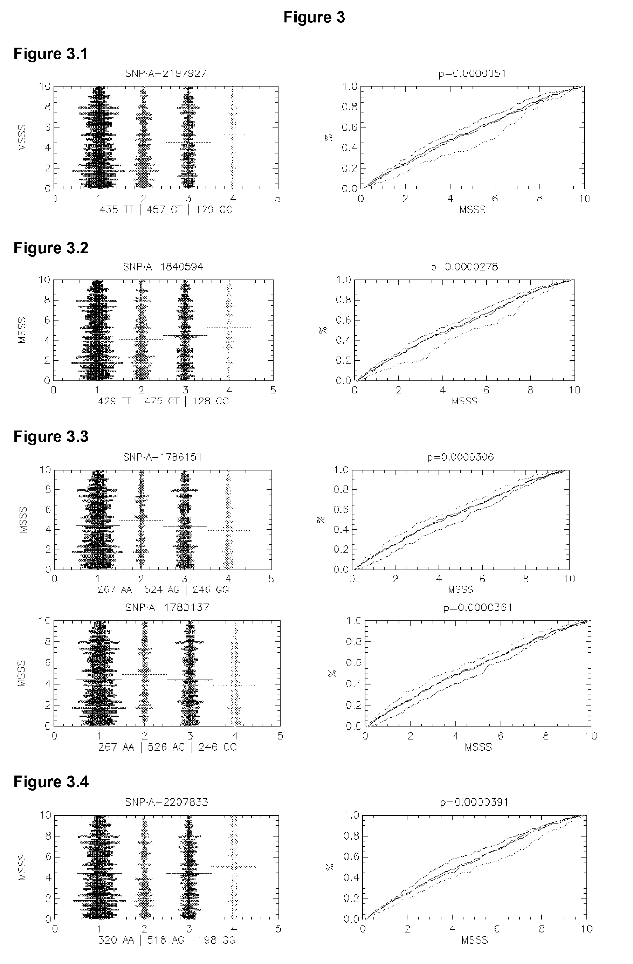

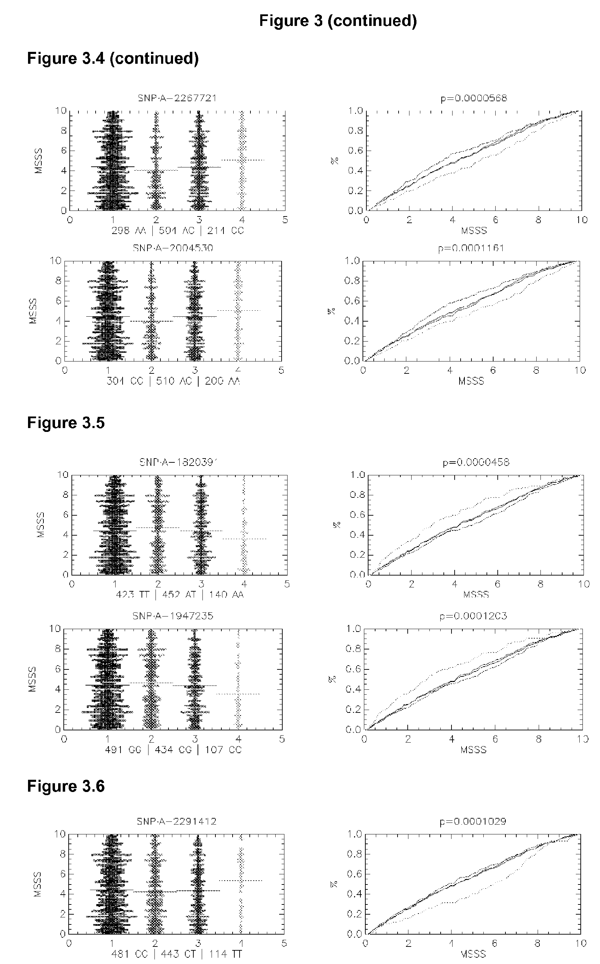

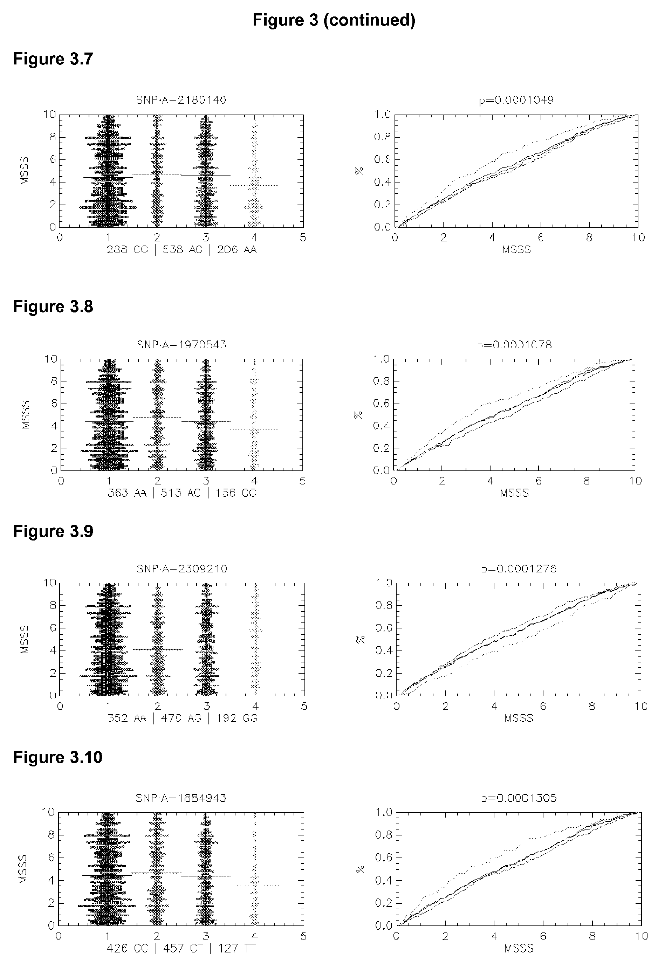

### Figure 3. Examples of SNP associated with disease severity.

The scatter plots on the left represent the MSSS distributions in the whole population (black: (1) far left column) and for the individuals having the major homozygote (red: (2) 2^{nd} column from left), the heterozygote (blue: 3^{rd} column from left) and the minor homozygote (green: far right column) for the considered SNP. Horizontal lines (resp. boxes) indicate MSSS averages (resp. standard deviations) within categories. Cumulative distribution functions are represented on the right.
**Figure 3****.****1****:** .SNP 1 desert chr4, rs6552511
**Figure 3****.****2****:** SNP 2 desert chr17, rs7221818
**Figure 3.3****:** SNPs 3 and 4. XYLT1, rs12927173 and rs2059283
**Figure 3****.****4****:** SNPs 5, 7 and 11. HIF1AN, rs1343522, rs4573623 and rs2495725
**Figure 3.5****:** SNPs 6 and 12. MGAT5, rs4953911 and rs3814022
**Figure 3.6****:** SNP 8. MEGF11, rs333548
**Figure 3.7****:** SNP 9. FGF14, rs10508075
**Figure 3.8****:** SNP 10. PDE9A, rs2839580
**Figure 3.9****:** SNP 13. MTPN, rs1078922
**Figure 3.10****:** SNP 14. CDH13, rs4315313

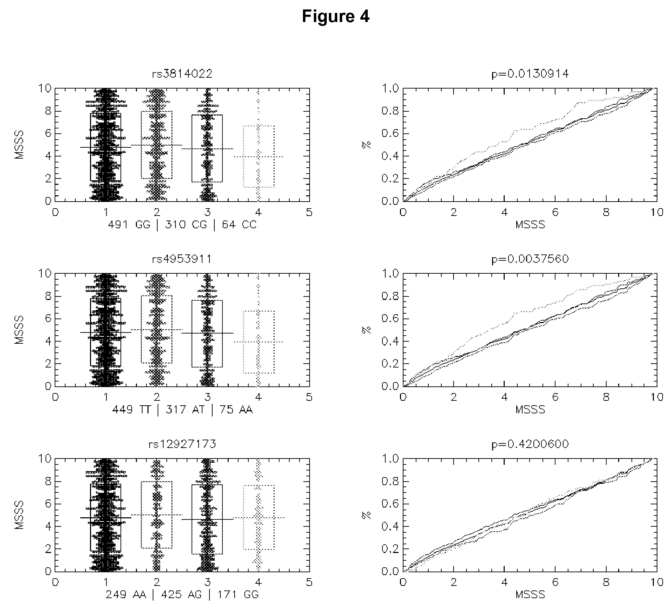

### Figure 4. Replication of SNPs in XYLT1 and MGAT5.

Association scatter plots (same legend as in Fig 3) of 3 SNPs on the replication dataset of 873 independent samples. The first two top SNPs are located in the MGAT5 gene and the third one is in the XYLT1 gene.

The SNPs and context sequences are depicted in the following

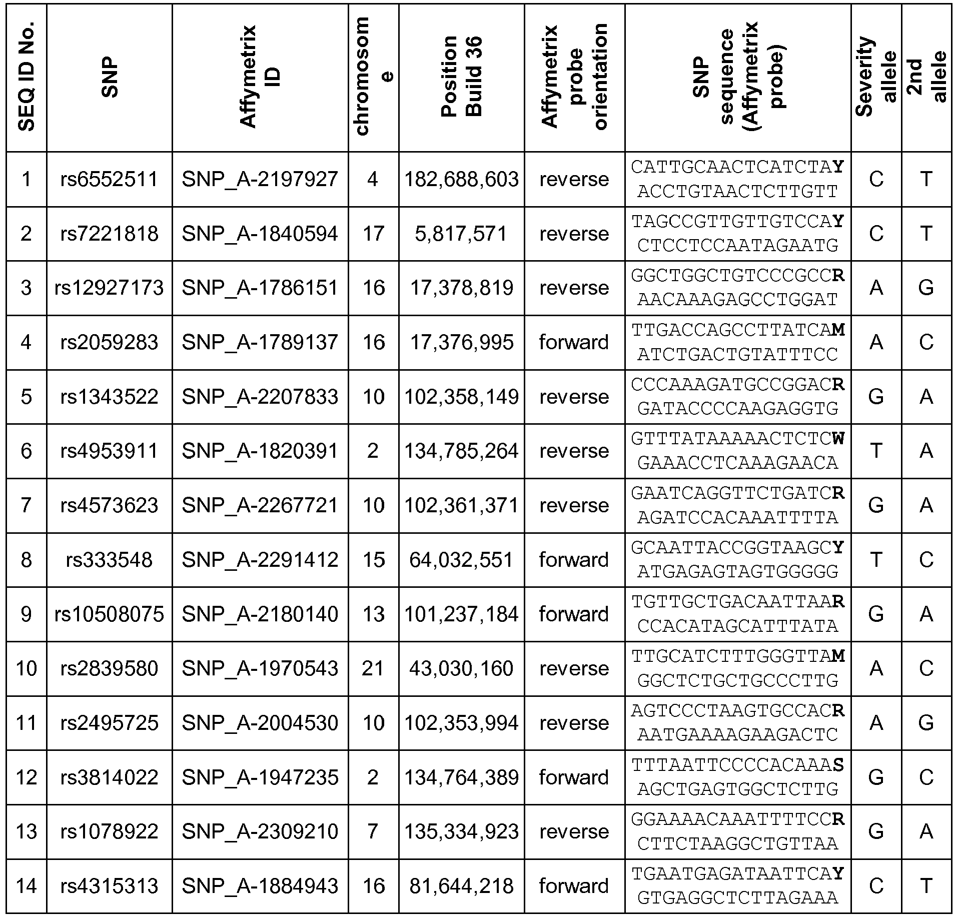

IUPAC SNP codes:

| **IUPAC Code** | **SNP** |
|---|---|
| R | G or A |
| Y | T or C |
| M | A or C |
| K | G or T |
| S | G or C |
| W | A or T |

Disclosed is a method for genotyping comprising the steps of a. using a nucleic acid isolated from a sample of an individual; and b. determining the type of nucleotide in SNP rs3814022, rs4953911, rs2059283, rs12927173, rs2495725, rs1343522, rs4573623 rs333548, rs10508075, rs2839580, rs2495725, rs3814022, rs1078922, and/or rs4315313 in one or both of the alleles of the diallelic marker, and /or in a SNPs in Linkage Disequilibrium (LD) with one or more of these SNPs.

SNPs of particular interest are preferably selected from rs3814022, rs4953911, rs2059283, rs12927173, rs2495725, rs1343522 and/or rs4573623.

Disclosed are SNPs in Linkage Disequilibrium (LD) with one or more of the identified SNPs, as expressed by a LD correlation coefficient *r*² greater than 0.8 in at least one population of at least 100 individuals, preferably a LD correlation coefficient *r*² greater than 0.95.

"Association" of a marker e.g. a SNP with the severity in a Multiple Sclerosis patient according to the invention means the statistically significant difference of marker frequencies between two populations of patients having different severity levels of Multiple Sclerosis.

"Severity" of Multiple Sclerosis (MS) may be expressed according to the invention with any means known in the field of MS like e.g. Expanded Disease Status Scale (EDSS) or with other commonly used techniques or measurements or definitions in the field. The term "residual disease activity" frequently used in this context and in the filed is to be understood as indicating a certain level of MS disease activity, e.g. showing clinical symptoms, as defined by any of the measurements or definitions usually applied in the field of MS. One indicator or measurement of "residual disease activity" can be the experience of relapse(s) or disease progression as e.g. measured by Expanded Disease Status Scale (EDSS) or Magnetic Resonance Imaging (MRI). As time frame one example is the assessment during two years of treatment. It is appreciated that other time frames may be defined and used, e.g. one year, three years, or others as usually applied in clinical study protocols and well known to the skilled person. The time frame of reference may be chosen so as to allow for a measurement and appropriate read-out. Equally applicable, other accepted disease status measurements may be applied as e.g. The Cambridge Multiple Sclerosis Basic Score (CAMBS) and others used by the skilled person. There exist various definitions of an MS attack in the field and as understood by the skilled person in the field of MS that may be applied according to the invention. Accordingly, various possibilities exist for the skilled person that can be applied when working the invention. Examples of the assessment or diagnosis of MS are published in Kurzke J.F., Neuroepidemiology, 1991, 10: 1 - 8 ; Kurzke J.F., Neurology, 1983, 33: 1444 - 1452 ; McDonald W.I et al., Ann. Neurol., 2001, 50: 121 - 127 ; Polman C.H. et al., Ann. Neurol. 2005, 58 : 840 - 846. Accordingly, a severity marker or SNP may represent a marker indicating high disease or low disease severity in a patient as compared to the MS population.

"Response" or "responders" to interferon treatment in an individual diagnosed as having MS, suffering from MS or a MS patient in the sense of the present invention is understood to be residual disease activity according to the criteria set out below upon interferon treatment, in particular with interferon-beta 1a or 1b, and in particular Rebif^{®}, Avonex^{®}, Cinnovex^{®} Betaseron^{®} and Extavia^{®}, of a MS patient. The response may be defined and/or measured as increase in time to the progression of the disease as measured by e.g. Expanded Disease Status Scale (EDSS) or with other commonly used techniques or measurements or definitions in the field. In particular it is to be understood as non-progression or non-worsening of MS or a stable clinical profile/activity or as the improvement of MS in e.g. clinical signs or measured with other means as e.g. MRI or CSF (cerebrospinal fluid) analysis. In particular it may be understood as less frequent relapses/attacks/exacerbation or milder relapses/attacks/exacerbation.
As used in the specification and the claims, "a" or "an" means one or more unless explicitly stated otherwise.
An "allele" is a particular form of a gene, genetic marker or other genetic locus, that is distinguishable from other forms of the gene, genetic marker or other genetic locus; e.g. without limitation by its particular nucleotide sequence. The term allele also includes for example without limitation one form of a single nucleotide polymorphism (SNP). An individual can be homozygous for a certain allele in diploid cells; i.e. the allele on both paired chromosomes is identical; or heterozygous for said allele; i.e. the alleles on both paired chromosomes are not identical.

A "genetic marker" is an identifiable polymorphic genetic locus. An example without limitation of a genetic marker is a single nucleotide polymorphism (SNP). A "marker" may be a genetic marker or any other marker, e.g. the expression level of a particular gene on nucleotide level as mRNA, useful in the context of the invention to be indicative of a response to interferon treatment.

A "genotype" as used herein refers to the combination of both alleles of a genetic marker, e.g. without limitation of an SNP, on a single genetic locus on paired (homologous) chromosomes in an individual. "Genotype" as used herein also refers to the combination of alleles of more than one genetic loci, e.g. without limitation of SNPs, on a pair or more than one pair of homologous chromosomes in an individual.

"Genotyping" is a process for determining a genotype of an individual.
"Locus" or "genetic locus" refers to a specific location on a chromosome or other genetic material.

"Oligonucleotide" refers to a nucleic acid or a nucleic acid derivative; including without limitation a locked nucleic acid (LNA), peptide nucleic acid (PNA) or bridged nucleic acid (BNA); that is usually between 5 and 100 contiguous bases in length, and most frequently between 5-40, 5-35, 5-30, 5-25, 5-20, 5-15, 5-10, 10-50, 10-40, 10-30, 10-25, 10-20, 15-50, 15-40, 15-30, 15-25, 15-20, 20-50, 20-40, 20-30 or 20-25 contiguous bases in length. The sequence of an oligonucleotide can be designed to specifically hybridize to any of the allelic forms of a genetic marker; such oligonucleotides are referred to as allele-specific probes. If the genetic marker is an SNP, the complementary allele for that SNP can occur at any position within an allele-specific probe. Other oligonucleotides useful in practicing the invention specifically hybridize to a target region adjacent to an SNP with their 3' terminus located one to less than or equal to about 10 nucleotides from the genetic marker locus, preferably ≤ about 5 nucleotides. Such oligonucleotides hybridizing adjacent to an SNP are useful in polymerase-mediated primer extension methods and are referred to herein as "primer-extension oligonucleotides." In a preferred embodiment, the 3'-terminus of a primer-extension oligonucleotide is a deoxynucleotide complementary to the nucleotide located immediately adjacent an SNP.

"Polymorphism" refers of two or more alternate forms (alleles) in a population of a genetic locus that differ in nucleotide sequence or have variable numbers of repeated nucleotide units. Polymorphisms occur in coding regions (exons), non-coding regions of genes or outside of genes. The different alleles of a polymorphism typically occur in a population at different frequencies, with the allele occurring most frequently in a selected population sometimes referenced as the "major" allele. Diploid organisms may be homozygous or heterozygous for the different alleles that exist. A diallelic polymorphism has two alleles. In said method preferably the identity of the nucleotides at said diallelic markers is determined for both copies of said diallelic markers present in said individual's genome. Any method known to the skilled person may be applied, preferably said determining is performed by a microsequencing assay. Furthermore, it is possible to amplify a portion of a sequence comprising the diallelic marker prior to said determining step, e.g. by PCR. However, any applicable method can be used.

It is now disclosed that the presence of a severity allele is characterized in rs3814022 by G, in rs4953911 by T, in rs2059283 by A, in rs12927173 by A, in rs2495725 by A, in rs1343522 by G, in rs4573623 by G, a T in rs333548, a G in rs10508075, a A in rs2839580, a A in rs2495725, a G in rs3814022, a G in rs1078922, and/or a C in rs4315313 and that it is indicative of the severity of the disease Multiple Sclerosis. In the particular SNP the respective base, A, T, C, G is present in one allele or preferably in both alleles and accordingly is indicative of the severity of MS. In particular, a SNP can indicate that an individual is probably more severly affected by MS or can represent a marker being indicative of being less severely affected by MS as compared to the average MS population.

The disclosure thus advantageously provides for a means to make a distinction between different patients and different patient groups of the overall MS population and in particular classify them according to severity of the disease. In this means state of the art molecular biology methods and apparatus are applied like PCR and PCR cyclers, and alghorithms of statistics generally known to the person skilled in the art. The patients may thus be grouped as to their expected MS severity according to the MSSS in e.g. very severe, medium severe, not very severe and slightly severe. The disclosure hence provides for a tool that has implications for handling such patients better according to their stage and severity of disease. In particular it now will be possible to adapt the treatment dosage and treatment scheme better on an individual patient level.

The disclosure relates to one or more SNPs selected from the group consisting of rs3814022, rs4953911, rs2059283, rs12927173, rs2495725, rs1343522, rs4573623, rs333548, rs10508075, rs2839580, rs2495725, rs3814022, rs1078922, rs4315313, SNPs in Linkage Disequilibrium (LD) with one or more of these SNPs for use in predicting the severity of the disease Multiple Sclerosis in an individual. The invention is directed to a method for predicting the severity of the disease Multiple Sclerosis in an individual as defined in claim 1.

The disclosed SNPs are selected from the group consisting of rs3814022, rs4953911, rs2059283, rs12927173, rs2495725, rs1343522, rs4573623, rs333548, rs10508075, rs2839580, rs2495725, rs3814022, rs1078922, rs4315313, SNPs in Linkage Disequilibrium (LD) with one or more of these SNPs.
SNPs of particular interest are preferably selected from rs3814022, rs4953911, rs2059283, rs12927173, rs2495725, rs1343522 and/or rs4573623.

Another aspect the invention relates Interferon-beta for use in a method for treating Multiple Sclerosis in an individual as defined in claim 7.

SNPs disclosed are rs3814022, rs4953911, rs2059283, rs12927173, rs2495725, rs1343522 and/or rs4573623.

The method may be used in particular advantageously to stratify and adjust the interferon dose and/or the time point of treatment. A possible measure may be a high dose treatment or a treatment before clinical signs of MS are visible in a patient identified as highly severe affected by MS. MRI may be applied to analyze a patient's disease status and a grouping/classification of the patient according to these results may be performed in a manner pointed out above. It will be particularly advantageous for an MS patient identified according to the invention to have a high risk to be a MS patient who will be severely affected by the disease, to be treated at an early time point in order to manage the disease early on. Thus, appropriate measures like an adequate interferon treatment and dosage can be chosen. In addition the awareness of the patient will support compliance with the treatment. An increased compliance has in turn positive effects on the treatment results as such and its efficacy.
Preferably the interferon-beta (IFN) is interferon-beta 1a or 1b. Examples of interferon-beta are Rebif^{®}, Avonex^{®}, Cinnovex^{®}, Betaseron^{®} or Extavia^{®}.
The dosage of IFN administered in the above method or use, as single or multiple doses, to an individual will vary in addition to the results of the patient grouping depending upon a variety of factors, including pharmacokinetic properties, the route of administration, patient conditions and characteristics (sex, age, body weight, health, size), extent of symptoms, concurrent treatments, frequency of treatment and the effect desired.
Standard dosages of human IFN-beta range from 80 000 IU/kg and 200 000 IU/kg per day or 6 MIU (million international units) and 12 MIU per person per day or 22 to 44 µg (microgram) per person. In accordance with the present invention, IFN may preferably be administered at a dosage of about 1 to 50 µg, more preferably of about 10 to 30 µg or about 10 to 20 µg per person per day.

The administration of active ingredients may be by intravenous, intramuscular or subcutaneous route. A preferred route of administration for IFN is the subcutaneous route.
IFN may also be administered daily or every other day, of less frequent. Preferably, IFN is administered one, twice or three times per week
A preferred route of administration is subcutaneous administration, administered e.g. three times a week. A further preferred route of administration is the intramuscular administration, which may e.g. be applied once a week.
Preferably 22 to 44 µg or 6 MIU to 12 MIU of IFN-beta is administered three times a week by subcutaneous injection. IFN-beta may be administered subcutaneously, at a dosage of 25 to 30 µg or 8 MIU to 9.6 MIU, every other day. 30 µg or 6 MIU IFN-beta may further be administered intramuscularly once a week.

### Examples

The following examples are not meant to be construed limiting for the invention. The following examples shall serve to illustrate the invention.

The examples show in a preferred embodiment of the invention the results of an approach to identify severity markers that is *(i)* genome-wide *(i.e.* hypothesis-free) and (ii) non categorical *(i.e.* continuous). First, three cohorts of MS patients (n = 1,040) were recruited from hospitals in France, Sweden and Italy, and genotyped for about 500,000 SNPs genome-wide with the Affymetrix Genechip® 500K technology. MS severity was continuously scored by MSSS, and correlation with genotypes of the most frequent polymorphisms (~ 105,000 SNPs) was evaluated by a non-parametric test between the MSSS distributions in patients homozygous for the alleles of each marker. The multiple-testing problem was controlled by False-Discovery Rate (FDR) estimation. The approach resulted in the identification of 14 severity markers, located in 8 different genes and 2 desert regions. Second, some markers have been genotyped on an independent replication cohort of 873 MS patients. Two glycosylation enzyme genes have been identified, thus supporting the importance of glycan regulation in MS.

### Materials & Methods

### Collections

A total number of 1,040 unrelated patients from France, Italy and Sweden were included in the 'screening' dataset and 873 unrelated and independent patients from France and Sweden in the 'replication' dataset (Table 1). All the subjects were Caucasians and had a diagnosis of Multiple Sclerosis according to McDonald's criteria ²⁷ and their disease courses were classified as either relapsing-remitting, secondary progressive or primary progressive ²⁸. Disability was scored using the Kurtzke EDSS. The mean age was 43.8 years, the mean EDSS score was 3.6 and the sex ratio was 2.1 females/males. Informed consent for the genetic analysis was obtained from all individuals and local ethical committees approved the study protocol.
The detailed demographic and clinical characteristics of MS patients are shown in Table 2 for the screening and replication datasets. The disease duration has been defined as the number of years between the year of onset of first symptom and the year of last examination with EDSS assessment, in most cases at entry in the study. The age at onset was defined as the first episode of neurological dysfunction suggestive of demyelinating disease.

### Grading of disability

The Kurtzke EDSS is the most widely used measure of disability in MS studies, but it does not take into account the disease duration, a parameter that is critical in describing the rate of progression. For this reason we used the MSSS¹⁴, which provides a measure for disease severity in an individual patient on a cross-sectional basis. This scale relates scores on the EDSS to the distribution of disability in a large dataset of patients with comparable disease durations. The MSSS is computed using the *MSSStest* software program v2.0 described in ¹⁴.

### Genotyping & quality control

DNA samples of the screening dataset have been studied independently using the Affymetrix GeneChip® human mapping 500K technology. Genotypes of the 497,641 SNPs selected by Affymetrix were called for each DNA sample with the B-RLMM software program, ensuring a minimal call rate of 97%. Only SNPs from autosomal chromosomes were kept for analysis. In order to avoid biases due to very low genotype frequencies, markers with low Minor Allele Frequency (MAF < 30%) or high rate of missing data (proportion of untyped DNA > 5%) were filtered out. We chose to focus on very frequent markers (MAF > 30%), which ensure a minimum minor homozygote frequency greater than 9% under Hardy-Weinberg equilibrium (and then a minor homozygote population size greater than 100 on average).
DNA samples of the replication dataset have been genotyped independently for selected SNPs using Applied BioSystems TaqMan® genotyping assay.

### Severity scan

For every SNP, a Wilcoxon rank-sum test ²⁹ was performed on the two sets of MS Severity Scores corresponding to patients homozygous for the alleles of each marker. This non-parametric test assigns a probability value (*p*-value) to every SNP. For the screening dataset, the False Discovery Rate (FDR) is estimated by permutation: *(i)* the null distribution is simulated by shuffling MS Severity Scores, recalculating Wilcoxon *p*-values, and repeating the process 10,000 times; (ii) the FDR is computed as follows for every p-value threshold α: FDR = min(1, *p.m* / *R),* where R is the number of positives at level α (number of SNPs with a *p*-value smaller than α), *m* is the number of tests performed (number of scanned SNPs), and *p* is the probability to have a *p*-value smaller than α under the null hypothesis, as estimated by the previous step of permutations^{30,31}.

### Genomic analysis

SNPs were located on the NCBI v36 human genome sequence. Gene structure (exons and introns) annotations were taken from ENSEMBL release 43³². Haplotypes and LD matrices were computed using HaploView³³ using the solid spine of LD method with a 0.8 *D*'extension cut-off.

### Results

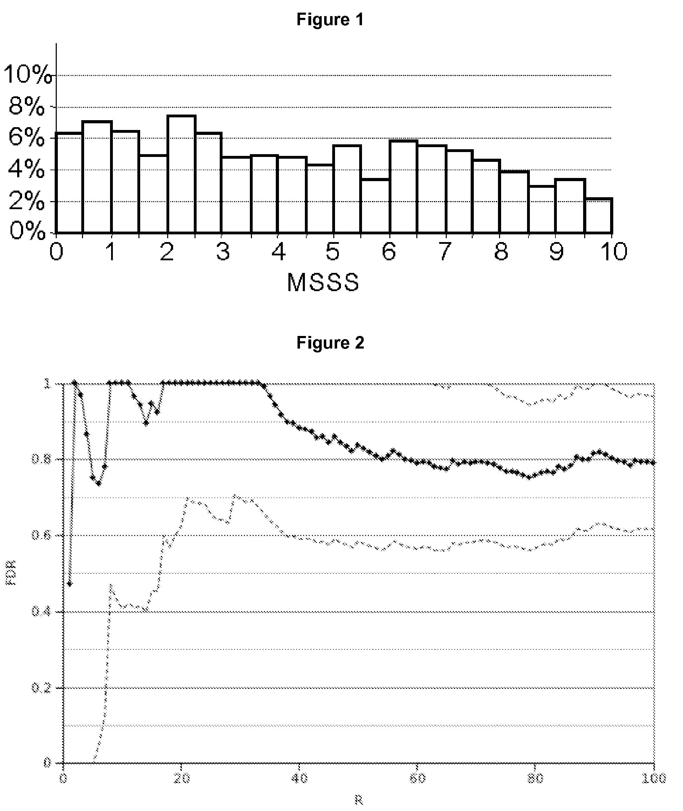

Over the 1,040 patients, the MSSS was on average 4.42 (standard deviation 2.79) spanning from 0.086 to 9.964 (see global distribution in **Figure 1**). Out of the 497,641 SNPs, 105,035 (21 %) survive the filtering criteria and are used for analysis, covering 63% of the genome. The FDR of observed results was estimated with 10,000 rounds of MSSS shuffling and plotted in **Figure 2** for the 100 smallest *p*-values. The FDR starts high (around 50%), rises quickly to an 80% plateau and then converges slowly towards 1. When considering the lower boundary of the 95% confidence interval, a 40% FDR threshold selected 14 SNPs (**Table 3**). These SNPs correspond to frequent genotypes (as ensured by the initial 30% MAF filter) and were all under Hardy-Weinberg Equilibrium. Selection corresponds to a severity *p*-value cut-off of 1.4e-4. The correlation between genotypes and MSSS is illustrated in **Figure 3**.

In classical categorical approaches, the MSSS scale is separated in categories, for instance mild and severe forms of MS, and classical association studies are performed to detect genotype differences between these two categories. When applied to our data set, using for instance two groups of 501 mild MS forms (MSSS < 4) and 356 severe MS forms (MSSS > 6), we failed to detect any significant associated SNP after multiple-testing correction by FDR ³¹. For instance, the SNP rs7221818 (ranked 2 in our continuous approach, see (Table 3) was ranked 67 in the categorical approach (genotypic *p*-value = 6.7e-4) and the FDR for this selection was estimated at 80%. Only the first-ranked SNP rs6552511 is retrieved by categorical approaches, using various MSSS thresholds (data not shown). Once these 14 SNPs have been selected by the continuous scan approach, it is however possible to analyze them in terms of classical categorical relative risks and odds ratios: 9 of the minor genotypes are associated with higher MSSS (relative risks range from 1.5 to 2.3) and 5 are associated with lower MSSS (risks range from 0.4 to 0.8, see tables for details).

These SNPs are mapped onto the human genome sequence and compared with gene annotations of ENSEMBL. Mapping details are presented in **Table 4**. Two SNPs (rs6552511 and rs7221818) are located in desert regions (the closest gene is located more than 100 kb away). The other 12 SNPs fall within or less than 100 kb away from 8 genes. Some of these genes (XYLT1, HIF1AN and MGAT5) are represented by several SNPs that define Linkage Disequilibrium (LD) severity blocks within genes. The three markers located 3' of HIF1AN on chromosome 10 are in a LD block that does not contain any part of the HIF1AN gene structure (the block is 50 kb away from the HIF1AN stop codon) or any known HIF1AN regulatory region. The rs1078922 SNP is located 22 kb 5' of the MTPN gene. Other SNPs fall in introns of the assigned genes: first intron of XYLT1 (2 SNPs), second intron of MGAT5 (2 SNPs), eighth intron of MEGF11, third intron of FGF14, seventh intron of PDE9A, and second intron of CDH13.

Signals in XYLT1 and MGAT5 were replicated because *(i)* those signals are represented by multiple SNPs in LD, which can be considered as a technical replication *per se* and *(ii)* these two genes encode for glycosylation enzymes and are biologically interesting candidates (see Discussion). Three SNPs were chosen in the two genes: rs12927173 in XYLT1, and rs3814022 and rs4953911 in MGAT5 (a second SNP, rs2059283, was chosen in XYLT1 but the manufacturer was unable to deliver primers). The *p*-values of these 3 SNPs in the replication dataset (*n* = 873) are respectively 0.42, 1.31e-2 and 3.76e-3 (Figure 4 and **Table 5**). The association with MS severity is then replicated in this independent dataset for MGAT5 SNPs. Overall *p*-values on both datasets are 2.81e-6 and 1.54e-7 for rs3814022 and rs4953911 respectively. For the SNP in XYLT1 (rs12927173), the association is not reproduced in the replication dataset (*p* = 0.42). However the overall *p*-value on both datasets is still significant (*p* = 1.88e-4).

We have performed a whole-genome scan analysis of over 1,000 MS patients in order to identify markers associated with disease severity. The overall process has led to the identification of 2 markers in un-annotated regions, 3 SNPs in a LD block close to the HIF1AN gene, 1 SNP in the 5' region of MTPN, and 8 markers inside 6 other genes. Three markers in two genes have been selected and genotyped in an independent replication population, leading to the confirmation of the association of MGAT5 with disease severity. We discuss here the clinical and methodological choices that have made these results possible, and then focus on the biological relevance of selected and replicated severity genes.

There is no consensus method for measuring progression in MS using single, cross-sectional assessments of disability. The MSSS has been recently developed as a powerful method for comparing disease progression in genetic association studies. It adjusts the widely accepted measure of disability, the EDSS, for disease duration comparing an individual's disability with the distribution of scores in cases having equivalent disease duration. The MSSS is potentially superior to the non-linear EDSS for statistical evaluations, as it combines EDSS and disease duration in one variable that is normally distributed. In our three populations, the MSSS distribution it is not homogeneous. This can be explained by different composition of the populations in terms of disease courses, and also by the known inter-observer variability (since the collections come from three different hospitals). This heterogeneity in disability measure assessments might have a significant impact on association results, especially if using arbitrary MSSS cut-off thresholds to define categories.

Previously published severity studies (of candidate genes) classically implement association tests between mild and severe MS sub-populations. In our case, similar categorical approaches using different MSSS thresholds have failed to detect any significantly associated marker. Using cut-off values on EDSS (or derived) scores is probably too arbitrary and inadequate for defining homogeneous severity subgroups, as EDSS only partially (and sometimes subjectively) reflects MS prognosis. With clinical scores, continuous approaches appear then more suitable. For the scan, we have chosen to discard heterozygotes and perform two-sample U-tests between MS patients that are homozygotous for every SNP. It has two theoretical advantages over a classical linear regression approach on 3 samples. First it does not assume that heterozygote patients have an intermediate MS severity (between the severity of the two homozygote groups), which would be the case in an additive model of severity risk. Our approach theoretically allows the detection of dominant or recessive transmission modes for the risk alleles. Second, the Wilcoxon rank-sum test is a non-parametric test: it applies for non-Gaussian MSSS distributions. As a counterpart, this method is probably underpowered for rare markers. We then focused on frequent markers (MAF > 30%) for which the frequency of the minor genotype is greater than 9% under Hardy-Weinberg Equilibrium and is well represented in our screened population (n > 100). This filtering dramatically reduced the number of analyzed SNPs (down to 105,035) while maintaining reasonable genome coverage (63%). Bigger sample size would be required to investigate less frequent markers with this method (*e.g*. 2,500 individuals for 20% markers, 10,000 for 10% markers). We can see *a posteriori* that the MSSS distribution in the whole population is not uniform and that generally the MSSS distributions per SNP genotypes is not Gaussian (Figure 1 and SNP examples Figure 3). Finally, it is important to take into account the multiple-testing problem. With conservative family-wise error rate estimation methods (like the Bonferroni correction), there is no SNP selected, meaning we are not able to select a set of markers for which we estimate there is no false-positives. We have preferred to use FDR estimation to control for the multiple-testing because it is more flexible (it allows for a given proportion of false-positives, not necessarily 0%) and it takes into account the dependency between markers ³¹.

The FDR-controlled approach has resulted in the selection of 14 markers. The two first-ranked SNPs display important minor genotype frequency differences between mild (MSSS<2) and severe (MSSS>8) clinical outcomes (relative risks are around 2.2) and are then markers of interest for MS severity. They are however located in unannotated genomic region and it is therefore impossible to make hypotheses on their functional impact on disease prognosis. Other SNPs fall inside or close to annotated genes. Among them, MGAT5 is of particular biological interest. The MGAT5 (also known as GNT-V) gene encodes the beta-1,6 *N*-acetyl-glucosaminyltransferase, an enzyme involved in the synthesis of beta-1,6 GlcNAc-branched N-linked glycans attached to cell surface and secreted glycoproteins. In mice, MGAT5 deficiency has a protective role in tumor growth³⁴ and is associated with enhanced susceptibility to experimental autoimmune encephalomyelitis (EAE) compared to wild-type ³⁵. The MGAT5 deficiency increases the number of T-cell receptors recruited to the antigen-presenting surface, thereby reducing the requirement for CD28 co-receptor engagement. CD28 and MGAT5 function as opposing regulators of T-cell activation thresholds and susceptibility to immune disease. Association of CD28 with MS severity had previously been tested and shown to be non significant ²¹. Moreover, the expression of beta-1,6 GlcNAc-branched N-linked glycans selectively inhibits Th1 cell differentiation and enhances the polarization of Th2 cells ³⁶. Deficient glycosylation has been also observed in lymphomonocytes from MS patients: a decrease of GCNT1 (another glucosaminyltransferase) activity by 25-30% is correlated with the occurrence of acute clinical phases of MS and the presence of active lesions in relapsing-remitting ³⁷. We therefore support here the association of MGAT5 and more generally of the GlcNAc-branched N-linked glycans with MS prognosis. Like MGAT5, XYLT1 (xylosyltransferase I, XT-I) is an enzyme implicated in glyscosylation. XYLT1 is the chain-initiating enzyme involved in the biosynthesis of glycosaminoglycan (GAG)-containing proteoglycans. Proteoglycans, a large group of glycoproteins, are of two main types, chondroitin sulfate (CSPGs) and heparin sulfate (HSPGs). Most CSPGs are secreted from cells and participate in the formation of the extracellular matrix (ECM). CSPGs are the most abundant type of proteoglycans expressed in the mammalian CNS and mainly act as barrier molecules affecting axon growth, cell migration and plasticity, particularly through their GAG-chains. A lesion to the adult CNS provokes the formation of a glial scar, which consists of proliferating and migrating glial cells (mainly reactive astrocytes, microglia and oligodendrocyte precursors) that upregulate several ECM molecules, including CSPGs. The proteoglycans of the glial scar might play a protective role, but the glial scar and its associated CSPGs are one of the main impediments to axon regeneration of injured CNS neurons³⁸. In MS, alteration of ECM molecules have been reported and excessive production and deposition of basement membrane constituents in active MS lesions have been shown and may contribute to axonal loss ³⁹. Thus, because XYLT1 initiates GAG-chain elongation and synthesis of CSPGs, two teams have developed a DNA enzyme which target
the mRNA of this enzyme and show a reduction of CSPGs ^{40,41}. In addition to this link with MS, XYLT1 has shown increased activity in the serum of patients with systemic sclerosis that correlates with clinical classification ⁴². Other genes assigned to the selected severity markers are HIF1AN (inhibitor of the Hypoxia-Inducible Factor 1 alpha), MEGF11 (multiple EGF-like domains 11), FGF14 (fibroblast growth factor 14), PDE9A (phosphodiesterase 9A), MTPN (myotrophin) and CDH13 (cadherin 13). No significant marker has been found in the previously reported regions associated with MS severity ²³⁻²⁶.

Because MGAT5 and XYLT1 are biologically relevant candidates that share similar glycosylation roles, we decided to replicate the experiment of an independent population. As a result, two SNPs in MGAT5 were clearly confirmed and one SNP in XYLT1 was not found associated in the replication dataset.

In conclusion, the first genome-wide MS severity scan we have performed has led to the hypothesis-free identification of markers associated with disease prognosis. The understanding of molecular mechanisms underlying the disease progression is a crucial point that needs to be addressed in parallel with the search for susceptibility factors. Two of the main identified genes, MGAT5 and XYLT1, are involved in glycosylation processes, thus confirming the importance of glycan regulation in MS. Among those two, MGAT5 was confirmed in an independent replication dataset, whereas XYLT1 replication led to more conflicting results in our study. Glycans play a pivotal role in modulating molecular interactions in the context of multiple physiologic systems, including immune-defense, and glycosylation has been shown to have a critical role in the overall regulation of the immune response ^{43,44}. Protein glycosylation is mechanistically important in the pathogenesis of autoimmune diseases: several evidences support the "Remnant Epitopes Generate Autoimmunity (REGA) model" in MS, rheumatoid arthritis (RA) and diabetes ⁴⁵. According to this model, the autoimmune process involves cytokines, chemokines and proteinases that cleave glycoproteins into remnant epitopes that are presented to autoreactive T lymphocytes, maintaining the autoimmune reaction. Examples of substrates yielding such remnant epitopes include myelin basic protein, αB-crystallin and interferon-β in MS, and type II collagen in RA ⁴⁶. The REGA model has been tested in vivo with the use of animal model and could have interesting therapeutic implications since inhibition of proteinases, such as gelatinase B for MS or RA, results in beneficial effects ^{47,48}.

### Tables

**Table 1. Multiple Sclerosis collections**

| **Dataset** | **Origin** | **#individuals** | **RR (%)** | **SP (%)** | **PP (%)** |
|---|---|---|---|---|---|
| Screening population | Rennes (France) | 384 | 172 (45%) | 135 (35%) | 77 (20%) |
| | Huddinge (Sweden) | 299 | 194 (65%) | 83 (28%) | 22 (7%) |
| | San Raffaele (Italy) | 357 | 228 (64%) | 94 (26%) | 35 (10%) |
| | Total | 1,040 | 594 (57%) | 312 (30%) | 134 (13%) |
| Replication population | Rennes (France) | 184 | 110 (60%) | 44 (24%) | 30 (16%) |
| | Huddinge (Sweden) | 689 | 277 (40%) | 348 (51%) | 61 (9%) |
| | Total | 873 | 387 (44%) | 392 (45%) | 91 (10%) |
| Overall total | | 1,913 | 981 (51%) | 704 (37%) | 225 (12%) |

**Table 2. Demographic and average clinical characteristics of MS patients in the screening and replication datasets**

| **Screening Population** | **MS (n=1040)** | **RR (n=594)** | **SP (n=312)** | **PP (n=134)** |
|---|---|---|---|---|
| Female/male | 704/336 | 427/167 | 201/111 | 76/58 |
| Age (years) | 43.2 | 38.9 | 48.4 | 50.2 |
| Disease duration (years) | 12.3 | 9.3 | 18.4 | 11.4 |
| Age at disease onset (years) | 30.9 | 29.6 | 30.0 | 38.8 |
| EDSS | 3.6 | 2.0 | 5.4 | 5.3 |

| **Replication Population** | **MS (n=879)** | **RR (n=387)** | **SP (n=392)** | **PP (n=91)** |
|---|---|---|---|---|
| Female/male | 631/242 | 298/89 | 248/144 | 52/39 |
| Age (years) | 52.5 | 45.8 | 57.9 | 57.9 |
| Disease duration (years) | 20.4 | 14.9 | 26.1 | 18.9 |
| Age at disease onset (years) | 32.1 | 30.9 | 31.8 | 39.0 |
| EDSS | 4.5 | 2.6 | 6.0 | 5.3 |

**Table 3. SNPs selected at 40% FDR lower-bound threshold.**

| **SNP id** | **rank** | **Homozygote 1** | | | | **Heterozygote** | | | | **Homozygote 2** | | | | **severity** |
|---|---|---|---|---|---|---|---|---|---|---|---|---|---|---|
| | | | *n* | ***m*** | *sd* | | *n* | ***m*** | *sd* | | *n* | ***m*** | *sd* | p-value |
| rs6552511 | 1 | TT | 435 | 4.03 | 2.69 | CT | 457 | 4.54 | 2.80 | CC | 129 | 5.35 | 2.77 | 5.11E-06 |
| rs7221818 | 2 | TT | 429 | 4.10 | 2.73 | CT | 475 | 4.51 | 2.82 | CC | 128 | 5.29 | 2.73 | 2.78E-05 |
| rs12927173 | 3 | AA | 267 | 4.96 | 2.70 | AG | 524 | 4.37 | 2.81 | GG | 246 | 3.94 | 2.75 | 3.06E-05 |
| rs2059283 | 4 | AA | 267 | 4.94 | 2.69 | AC | 526 | 4.39 | 2.82 | CC | 246 | 3.94 | 2.75 | 3.61 E-05 |
| rs1343522 | 5 | AA | 320 | 4.01 | 2.71 | AG | 518 | 4.43 | 2.73 | GG | 198 | 5.08 | 2.93 | 3.91E-05 |
| rs4953911 | 6 | TT | 423 | 4.70 | 2.85 | AT | 452 | 4.42 | 2.72 | AA | 140 | 3.60 | 2.70 | 4.58E-05 |
| rs4573623 | 7 | AA | 298 | 4.06 | 2.75 | AG | 504 | 4.37 | 2.74 | GG | 214 | 5.08 | 2.86 | 5.68E-05 |
| rs333548 | 8 | CC | 481 | 4.25 | 2.78 | CT | 443 | 4.36 | 2.76 | TT | 114 | 5.39 | 2.80 | 1.03E-04 |
| rs10508075 | 9 | GG | 288 | 4.73 | 2.85 | AG | 538 | 4.55 | 2.79 | AA | 206 | 3.74 | 2.63 | 1.05E-04 |
| rs2839580 | 10 | AA | 363 | 4.78 | 2.86 | AC | 513 | 4.39 | 2.74 | CC | 156 | 3.73 | 2.66 | 1.08E-04 |
| rs2495725 | 11 | GG | 304 | 3.99 | 2.69 | AG | 510 | 4.44 | 2.74 | AA | 200 | 5.05 | 2.95 | 1.16E-04 |
| rs3814022 | 12 | GG | 491 | 4.65 | 2.82 | CG | 434 | 4.39 | 2.74 | CC | 107 | 3.53 | 2.68 | 1.20E-04 |
| rs1078922 | 13 | AA | 352 | 4.09 | 2.68 | AG | 470 | 4.40 | 2.82 | GG | 192 | 5.03 | 2.81 | 1.28E-04 |
| rs4315313 | 14 | CC | 426 | 4.70 | 2.83 | CT | 457 | 4.41 | 2.75 | TT | 127 | 3.60 | 2.72 | 1.30E-04 |

| | | | | | | | | | | | | | | |
|---|---|---|---|---|---|---|---|---|---|---|---|---|---|---|
| *n*: number of individuals having this genotype; *m* and *sd*: MSSS average and standard deviation for these people. The *p*-value refers to the rank-sum test performed on homozgygote categories (heterozygotes are not used, see text). | | | | | | | | | | | | | | |

**Table 4. Genomic location of selected severity SNPs.**

| ***SNP id*** | **rank** | **chromosome** | **position** | **MAF** | **Closest gene** |
|---|---|---|---|---|---|
| rs6552511 | 1 | 4q34 | 182,688,603 | 35% | *(desert)* |
| rs7221818 | 2 | 17p13 | 5,742,055 | 35% | *(desert)* |
| rs12927173 | 3 | 16p13.1 | 17,378,835 | 49% | XYLT1 *(intron)* |
| rs2059283 | 4 | 16p13.1 | 17,377,011 | 49% | XYLT1 *(intron)* |
| rs1343522 | 5 | 10q24 | 102,358,165 | 44% | 58kb 3' of HIF1AN |
| rs4953911 | 6 | 2q21 | 134,785,280 | 36% | MGAT5 *(intron)* |
| rs4573623 | 7 | 10q24 | 102,361,387 | 46% | 61kb 3' of HIF1AN |
| rs333548 | 8 | 15q22 | 64,032,567 | 32% | MEGF11 *(intron)* |
| rs10508075 | 9 | 13q32 | 101,237,200 | 46% | FGF14 *(intron)* |
| rs2839580 | 10 | 21q22 | 43,030,176 | 40% | PDE9A *(intron)* |
| rs2495725 | 11 | 10q24 | 102,354,010 | 45% | 54kb 3' of HIF1AN |
| rs3814022 | 12 | 2q21 | 134,764,405 | 31% | MGAT5 *(intron)* |
| rs1078922 | 13 | 7q33 | 135,334,939 | 42% | 22kb 5' of MTPN |
| rs4315313 | 14 | 16q23 | 81,644,234 | 35% | CDH13 *(intron)* |

**Table 5. Replication of severity markers in independent samples**

| **SNP** | **Dataset** | **Major homozygote** | | | **Heterozygote** | | | **Minor homozygote** | | | **p-value** |
|---|---|---|---|---|---|---|---|---|---|---|---|
| | | **# samples** | **MSSS mean** | **MSSS sd** | **# samples** | **MSSS mean** | **MSSS sd** | **# samples** | **MSSS mean** | **MSSS sd** | |
| rs3814022 | Screen | 491 | 4.65 | 2.82 | 434 | 4.39 | 2.74 | 107 | 3.53 | 2.68 | 1.20E-04 |
| | Replic. | 491 | 4.99 | 2.99 | 310 | 4.66 | 2.99 | 64 | 3.97 | 2.72 | 1.31E-02 |
| | Total | 982 | 4.82 | 2.91 | 744 | 4.50 | 2.84 | 171 | 3.69 | 2.70 | 2.81 E-06 |
| rs4953911 | Screen | 423 | 4.70 | 2.85 | 452 | 4.42 | 2.72 | 140 | 3.60 | 2.70 | 4.58E-05 |
| | Replic. | 449 | 5.06 | 2.99 | 317 | 4.69 | 2.99 | 75 | 3.95 | 2.77 | 3.76E-03 |
| | Total | 872 | 4.88 | 2.93 | 769 | 4.53 | 2.84 | 215 | 3.72 | 2.72 | 1.54E-07 |
| rs1292717 3 | Screen | 267 | 4.96 | 2.70 | 524 | 4.37 | 2.81 | 246 | 3.94 | 2.75 | 3.06E-05 |
| | Replic. | 249 | 5.01 | 2.95 | 425 | 4.66 | 3.07 | 171 | 4.80 | 2.83 | 0.42 |
| | Total | 516 | 4.98 | 2.82 | 949 | 4.50 | 2.93 | 417 | 4.29 | 2.81 | 1.88E-04 |

### References

1. Dyment DA, Ebers GC, Sadovnick AD. Genetics of multiple sclerosis. Lancet Neurol. 2004; 3:104-110
2. Hafler DA, Compston A, Sawcer S et al. Risk alleles for multiple sclerosis identified by a genomewide study. N Engl J Med. 2007; 357
3. Lincoln MR, Montpetit A, Cader MZ et al. A predominant role for the HLA class II region in the association of the MHC region with multiple sclerosis. Nat Genet. 2005; 37:1108-1112
4. Compston A, Sawcer S. Genetic analysis of multiple sclerosis. Curr Neurol Neurosci Rep. 2002; 2:259-266
5. Fogdell-Hahn A, Ligers A, Gronning M et al. Multiple sclerosis: a modifying influence of HLA class I genes in an HLA class II associated autoimmune disease. Tissue Antigens. 2000; 55:140-148
6. Harbo HF, Lie BA, Sawcer S et al. Genes in the HLA class I region may contribute to the HLA class II-associated genetic susceptibility to multiple sclerosis. Tissue Antigens. 2004; 63:237-247
7. Yeo TW, De Jager PL, Gregory SG et al. A second major histocompatibility complex susceptibility locus for multiple sclerosis. Ann Neurol. 2007; 61:228-236
8. Gregory SG, Schmidt S, Seth P et al. Interleukin 7 receptor alpha chain (IL7R) shows allelic and functional association with multiple sclerosis. Nat Genet. 2007;
9. Zhang Z, Duvefelt K, Svensson F et al. Two genes encoding immune-regulatory molecules (LAG3 and IL7R) confer susceptibility to multiple sclerosis. Genes Immun. 2005; 6:145-152
10. Lundmark F, Duvefelt K, Hillert J. Genetic association analysis of the interleukin 7 gene (IL7) in multiple sclerosis. J Neuroimmunol. 2007; 192:171-173
11. Hensiek AE, Seaman SR, Barcellos LF et al. Familial effects on the clinical course of multiple sclerosis. Neurology. 2007; 68:376-383
12. Rasmussen HB, Clausen J. Genetic risk factors in multiple sclerosis and approaches to their identification. J Neurovirol. 2000; 6 Suppl 2:S23-S27
13. Kurtzke JF. Rating neurologic impairment in multiple sclerosis: an expanded disability status scale (EDSS). Neurology. 1983; 33:1444-1452
14. Roxburgh RH, Seaman SR, Masterman T et al. Multiple Sclerosis Severity Score: using disability and disease duration to rate disease severity. Neurology. 2005; 64:1144-1151
15. Kantarci OH, de AM, Weinshenker BG. Identifying disease modifying genes in multiple sclerosis. J Neuroimmunol. 2002; 123:144-159
16. Burwick RM, Ramsay PP, Haines JL et al. APOE epsilon variation in multiple sclerosis susceptibility and disease severity: some answers. Neurology. 2006; 66:1373-1383
17. Santos M, do Carmo CM, Edite RM et al. Genotypes at the APOE and SCA2 loci do not predict the course of multiple sclerosis in patients of Portuguese origin. Mult Scler. 2004; 10:153-157
18. Lindquist S, Schott BH, Ban M et al. The BDNF-Val66Met polymorphism: implications for susceptibility to multiple sclerosis and severity of disease. J Neuroimmunol. 2005; 167:183-185
19. Kroner A, Vogel F, Kolb-Maurer A et al. Impact of the Asp299Gly polymorphism in the toll-like receptor 4 (tlr-4) gene on disease course of multiple sclerosis. J Neuroimmunol. 2005; 165:161-165
20. Hensiek AE, Roxburgh R, Meranian M et al. Osteopontin gene and clinical severity of multiple sclerosis. J Neurol. 2003; 250:943-947
21. van VT, Crusius JB, van WL et al. CTLA-4 and CD28 gene polymorphisms in susceptibility, clinical course and progression of multiple sclerosis. J Neuroimmunol. 2003; 140:188-193
22. Schreiber K, Otura AB, Ryder LP et al. Disease severity in Danish multiple sclerosis patients evaluated by MRI and three genetic markers (HLA-DRB1*1501, CCR5 deletion mutation, apolipoprotein E). Mult Scler. 2002; 8:295-298
23. Mann CL, Davies MB, Stevenson VL et al. Interleukin 1 genotypes in multiple sclerosis and relationship to disease severity. J Neuroimmunol. 2002; 129:197-204
24.Almeras L, Meresse B, Seze J et al. Interleukin-10 promoter polymorphism in multiple sclerosis: association with disease progression. Eur Cytokine Netw. 2002; 13:200-206
25. Goertsches R, Comabella M, Navarro A et al. Genetic association between polymorphisms in the ADAMTS14 gene and multiple sclerosis. J Neuroimmunol. 2005; 164:140-147
26. Mead RJ, Neal JW, Griffiths MR et al. Deficiency of the complement regulator CD59a enhances disease severity, demyelination and axonal injury in murine acute experimental allergic encephalomyelitis. Lab Invest. 2004; 84:21-28
27. McDonald WI, Compston A, Edan G et al. Recommended diagnostic criteria for multiple sclerosis: guidelines from the International Panel on the diagnosis of multiple sclerosis. Ann Neurol. 2001; 50:121-127
28. Lublin FD, Reingold SC. Defining the clinical course of multiple sclerosis: results of an international survey. National Multiple Sclerosis Society (USA) Advisory Committee on Clinical Trials of New Agents in Multiple Sclerosis. Neurology. 1996; 46:907-911
29. Wilcoxon F. Individual comparisons by ranking methods. Biometrics. 1945; 1:80-83
30. Storey JD, Tibshirani R. Statistical significance for genomewide studies. Proc Natl Acad Sci USA. 2003; 100:9440-9445
31. Forner, K., Lamarine, M., Guedj, M., Dauvillier, J., and Wojcik, J. Universal false discovery rate estimation methodology for genome-wide association studies. Human Heredity 2007. Ref Type: In Press
32. Birney E. Ensembl 2007. Nucleic Acids Res. 2007; 35:610-617
33. Barrett JC, Fry B, Maller J et al. Haploview: analysis and visualization of LD and haplotype maps. Bioinformatics. 2005; 21:263-265
34. Granovsky M, Fata J, Pawling J et al. Suppression of tumor growth and metastasis in Mgat5-deficient mice. Nat Med. 2000; 6:306-312
35. Demetriou M, Granovsky M, Quaggin S et al. Negative regulation of T-cell activation and autoimmunity by Mgat5 N-glycosylation. Nature. 2001; 409:733-739
36. Morgan R, Gao G, Pawling J et al. N-acetylglucosaminyltransferase V (Mgat5)-mediated N-glycosylation negatively regulates Th1 cytokine production by T cells. J Immunol. 2004; 173:7200-7208
37.Orlacchio A, Sarchielli P, Gallai V et al. Activity levels of a beta1,6 N-acetylglucosaminyltransferase in lymphomonocytes from multiple sclerosis patients. J Neurol Sci. 1997; 151:177-183
38. Carulli D, Laabs T, Geller HM et al. Chondroitin sulfate proteoglycans in neural development and regeneration. Curr Opin Neurobiol. 2005; 15:116-120
39. van HJ, Bo L, Dijkstra CD et al. Extensive extracellular matrix depositions in active multiple sclerosis lesions. Neurobiol Dis. 2006; 24:484-491
40. Grimpe B, Silver J. A novel DNA enzyme reduces glycosaminoglycan chains in the glial scar and allows microtransplanted dorsal root ganglia axons to regenerate beyond lesions in the spinal cord. J Neurosci. 2004; 24:1393-1397
41. Grimpe B, Pressman Y, Lupa MD et al. The role of proteoglycans in Schwann cell/astrocyte interactions and in regeneration failure at PNS/CNS interfaces. Mol Cell Neurosci. 2005; 28:18-29
42. Gotting C, Sollberg S, Kuhn J et al. Serum xylosyltransferase: a new biochemical marker of the sclerotic process in systemic sclerosis. J Invest Dermatol. 1999; 112:919-924
43. Garcia GG, Berger SB, Sadighi Akha AA et al. Age-associated changes in glycosylation of CD43 and CD45 on mouse CD4 T cells. Eur J Immunol. 2005; 35:622-631
44. Grabie N, Delfs MW, Lim YC et al. Beta-galactoside alpha2,3-sialyltransferase-I gene expression during Th2 but not Th1 differentiation: implications for core2-glycan formation on cell surface proteins. Eur J Immunol. 2002; 32:2766-2772
45. Descamps FJ, Van den Steen PE, Nelissen I et al. Remnant epitopes generate autoimmunity: from rheumatoid arthritis and multiple sclerosis to diabetes. Adv Exp Med Biol. 2003; 535:69-77
46. Opdenakker G, Dillen C, Fiten P et al. Remnant epitopes, autoimmunity and glycosylation. Biochim Biophys Acta. 2006; 1760:610-615
47. Dubois B, Masure S, Hurtenbach U et al. Resistance of young gelatinase B-deficient mice to experimental autoimmune encephalomyelitis and necrotizing tail lesions. J Clin Invest. 1999; 104:1507-1515
48. Itoh T, Matsuda H, Tanioka M et al. The role of matrix metalloproteinase-2 and matrix metalloproteinase-9 in antibody-induced arthritis. J Immunol. 2002; 169:2643-2647
49. Kurzke J.F., Neuroepidemiology, 1991, 10: 1 - 8
50. Kurzke J.F., Neurology, 1983, 33: 1444 - 1452
51. McDonald W.I et al., Ann. Neurol., 2001, 50: 121 - 127
52. Polman C.H. et al., Ann. Neurol. 2005, 58 : 840 - 846

## Claims

1. A method for predicting the severity of the disease Multiple Sclerosis in an individual comprising the steps of:
a. using a nucleic acid isolated from a sample of an individual;
b. determining the type of nucleotide in SNP rs2059283 and/or rs12927173, in one or both alleles of the diallelic marker; and
c. correlating the result of the genotyping steps with the severity of the disease Multiple Sclerosis.

2. Method according to claim 1 wherein the identity of the nucleotides at said diallelic markers is determined for both copies of said diallelic markers present in said individual's genome.

3. Method according to any of claims 1 or 2 wherein said determining is performed by a microsequencing assay.

4. Method according to any of claims 1 to 3 further comprising amplifying a portion of a sequence comprising the diallelic marker prior to said determining step.

5. Method according to claim 4 wherein said amplifying is performed by PCR.

6. Method according to any one of claims 1 to 6, wherein the presence an A in rs2059283 and/or an A in rs12927173 indicates that the disease Multiple Sclerosis is severe in said individual.

7. Interferon-beta for use in the treatment of Multiple Sclerosis in a patient that has the allele A in rs2059283 and/or an A in rs12927173 wherein the treatment comprises the determination in-vitro of whether or not the patient has the said alleles.

8. The use according to claim 9 wherein the interferon-beta is interferon-beta 1 a or 1 b.

9. The method or use according to claim 9 wherein the interferon-beta is Rebif®, Avonex®, Cinnovex®, Betaseron® or Extavia®.

## Patentansprüche

1. Verfahren zur Vorhersage der Schwere der Krankheit Multiple Sklerose in einem Individuum, umfassend die folgenden Schritte:
a. Verwenden einer Nucleinsäure, isoliert aus einer Probe von einem Individuum,
b. Bestimmen des Typs des Nucleotids in SNP rs2059283 und/oder rs12927173, in einem oder beiden Allelen des di-allelischen Markers, und
c. Korrelieren des Ergebnisses der Genotypisierungsschritte mit der Schwere der Krankheit Multiple Sklerose.

2. Verfahren gemäß Anspruch 1, wobei die Identität der Nucleotide an den diallelischen Markern für beide Kopien der diallelischen Marker, die in dem Genom des Individuums vorliegen, bestimmt wird.

3. Verfahren gemäß einem der Ansprüche 1 oder 2, wobei das Bestimmen durch einen Mikrosequenzierungsassay durchgeführt wird.

4. Verfahren gemäß einem der Ansprüche 1 bis 3, ferner umfassend Amplifizieren eines Teils einer Sequenz, umfassend den diallelischen Marker, vor dem Bestimmungsschritt.

5. Verfahren gemäß Anspruch 4, wobei das Amplifizieren durch PCR durchgeführt wird.

6. Verfahren gemäß einem der Ansprüche 1 bis 6, wobei das Vorliegen eines A in rs2059283 und/oder eines A in rs12927173, anzeigt, dass die Krankheit Multiple Sklerose bei dem Individuum schwer ist.

7. Interferon-beta zur Verwendung in der Behandlung von Multipler Sklerose bei einem Patienten, welcher das Allel A in rs2059283 und/oder ein A in rs12927173 besitzt, wobei die Behandlung die Bestimmung in vitro umfasst, ob der Patient diese Allele besitzt, oder nicht.

8. Verwendung gemäß Anspruch 9, wobei das Interferon-beta Interferon-beta la oder 1b ist.

9. Verfahren oder Verwendung gemäß Anspruch 9, wobei das Interferon-beta Reif^{®}, Avonex^{®}, Cinnovex^{®}, Betaseron^{®} oder Extavia^{®} ist.

## Revendications

1. Procédé permettant de prédire la gravité d'une sclérose en plaques chez un individu, lequel procédé comporte les étapes suivantes :
a) prendre un acide nucléique isolé à partir d'un échantillon provenant d'un individu,
b) déterminer la nature du nucléotide au niveau du ou des site(s) SNP rs2059283 et/ou rs12927173, chez un seul allèle du marqueur biallélique ou chez les deux,
c) et corréler le résultat des étapes de génotypage avec la gravité de la maladie de sclérose en plaques.

2. Procédé conforme à la revendication 1, dans lequel l'identité des nucléotides au niveau desdits marqueurs bialléliques est déterminée pour les deux copies desdits marqueurs bialléliques présentes dans le génome dudit individu.

3. Procédé conforme à l'une des revendications 1 et 2, dans lequel ladite étape de détermination est réalisée par microséquençage.

4. Procédé conforme à l'une des revendications 1 à 3, qui comporte en outre, avant ladite étape de détermination, l'amplification d'une partie d'une séquence comprenant le marqueur biallélique.

5. Procédé conforme à la revendication 4, dans lequel ladite amplification est réalisée par PCR.

6. Procédé conforme à l'une des revendications 1 à 6, dans lequel la présence d'un nucléotide A au niveau du site rs2059283 et/ou d'un nucléotide A au niveau du site rs12927173 indique que chez ledit individu, la sclérose en plaques est grave.

7. Interféron-β pour utilisation dans le traitement de la sclérose en plaques chez un patient qui est porteur de l'allèle A au niveau du site rs2059283 et/ou de l'allèle A au niveau du site rs12927173, étant entendu que ledit traitement comporte le fait de déterminer in vitro si, oui ou non, le patient porte lesdits allèles.

8. Utilisation conforme à la revendication 9, dans laquelle l'interféron-β est de l'interféron-β 1a ou de l'interféron-β 1b.

9. Procédé ou utilisation conforme à la revendication 9, dans lequel ou laquelle l'interféron-β est du Rebif®, de l'Avonex®, du Cinnovex®, du Betaseron®, ou de l'Extavia®.
